(19) Europäisches Patentamt
European Patent Office
Office européen des brevets

(11) Numéro de publication : **0 370 867 B1**

(12) # FASCICULE DE BREVET EUROPEEN

(45) Date de publication du fascicule du brevet : **07.01.93 Bulletin 93/01**

(51) Int. Cl.$^5$ : **C07C 309/24,** A61K 7/42

(21) Numéro de dépôt : **89403146.7**

(22) Date de dépôt : **16.11.89**

(54) **Sels de métaux polyvalents de dérivés sulfonés du benzylidène-camphre et leur utilisation pour la protection de la peau contre le rayonnement ultraviolet.**

(30) Priorité : **22.11.88 LU 87394**

(43) Date de publication de la demande : **30.05.90 Bulletin 90/22**

(45) Mention de la délivrance du brevet : **07.01.93 Bulletin 93/01**

(84) Etats contractants désignés : **AT DE NL**

(56) Documents cités :
**FR-A- 2 236 515**
**FR-A- 2 383 904**
**FR-A- 2 528 420**

(73) Titulaire : **L'OREAL**
**14, Rue Royale**
**F-75008 Paris (FR)**

(72) Inventeur : **Lang, Gérard**
**44, Avenue Lacour**
**F-95210 Saint-Gratien (FR)**
Inventeur : **Forestier, Serge**
**16, Allée Ferdinand Buisson**
**F-77410 Claye-Souilly (FR)**
Inventeur : **Moire, Claudine**
**67, Allée Pluton**
**F-93600 Aulnay-sous-Bois (FR)**
Inventeur : **Lagrange, Alain**
**29, Rue Auguste Renoir**
**F-78400 Chatou (FR)**

(74) Mandataire : **Casalonga, Axel et al**
**BUREAU D.A. CASALONGA - JOSSE**
**Morassistrasse 8**
**W-8000 München 5 (DE)**

EP 0 370 867 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention est relative à de nouveaux sels de métaux polyvalents de dérivés sulfonés du benzylidène-camphre et à leur utilisation en tant que filtres solaires dans des compositions cosmétiques.

On sait que les radiations lumineuses de longueurs d'onde comprises entre 280 et 400 nm permettent le brunissement de l'épiderme humain et que les rayons de longueurs d'onde comprises entre 280 et 320 nm connus sous la dénomination "UV-B" provoquent également des érythèmes et des brûlures cutanés qui peuvent nuire au développement du bronzage.

On connaît déjà l'utilisation de composés actifs dans la zone de longueurs d'onde 280-320 nm précitée. Les brevets français n° 2 282 426 et 2 236 515 décrivent par exemple des sels de métaux polyvalents de dérivés du benzylidène-camphre sulfonés sur le radical méthyle en position 10 du camphre ou en position 3′ ou 4′ sur le noyau benzénique.

Toutefois, si les rayons UV-B de longueurs d'onde comprises entre 280 et 320 nm jouent un rôle prépondérant dans la production d'érythèmes solaires et doivent être filtrés, il n'en est pas moins vrai que les rayons UV-A de longueurs d'onde comprises entre 320 et 400 nm provoquant le brunissement de la peau, provoquent également une altération de celle-ci, notamment dans le cas d'une peau sensible ou d'une peau continuellement exposée au rayonnement solaire. On a constaté que les rayons UV-A peuvent potentialiser l'action des rayons UV-B. Les rayons UV-A favorisent le déclenchement de la réaction érythémateuse ou amplifient cette réaction chez certains sujets. De même, ils peuvent être à l'origine de réactions phototoxiques ou photoallergiques.

Par conséquent, on a recherché des composés susceptibles d'absorber aussi bien les rayons UV-A que les rayons UV-B nocifs pour la peau et d'être en mesure de protéger les produits sensibles à ces radiations.

Le brevet FR n° 2 528 420 décrit de tels composés, constitués par des sels de métaux alcalins ou d'amines, solubles dans l'eau, de dérivés du benzylidène-camphre sulfonés en position 10 du camphre.

Malheureusement, tous ces sels de métaux polyvalents ou de métaux alcalins de dérivés du benzylidène-camphre de l'art antérieur ont un pouvoir absorbant relativement faible lorsqu'ils sont utilisés à faible concentration. Pour obtenir une protection élevée, il faut donc soit augmenter leur concentration, soit ajouter d'autres filtres ou encore des pigments comme l'oxyde de titane.

Or, on sait que les agents filtrant les rayons UV peuvent provoquer des effets secondaires défavorables et qu'il est de l'intérêt du cosmétologue d'obtenir l'indice de protection souhaité avec la quantité la plus faible possible d'agents filtrants dans la composition.

Par ailleurs, lorsqu'on utilise un pigment, on obtient une composition cosmétique ayant un bon pouvoir filtrant, mais qui laisse subsister sur la peau, après application, une pellicule blanchâtre peu appréciée des utilisateurs.

On exige également d'une composition cosmétique filtrante, outre un indice de protection élevé et une application esthétique, qu'elle ne colle pas au toucher et qu'elle présente une bonne stabilité chimique et photochimique et également une grande persistance.

La persistance peut être définie comme la stabilité de l'indice de protection de la composition filtrante au cours de l'exposition du sujet au soleil. Il est important que cette persistance soit élevée car il est nécessaire que l'indice de protection soit constant au cours de l'exposition, ce qui permet d'éviter des applications répétées à intervalles réguliers et rapprochés afin d'obtenir une protection efficace de la peau contre les rayons UV.

Cet indice de protection peut varier soit parce que le filtre est instable photochimiquement, soit parce qu'il pénètre dans la peau et ne joue plus son rôle, ou encore parce qu'il s'élimine au cours de la baignade.

La demanderesse a découvert qu'une famille particulière de sels de métaux polyvalents de dérivés sulfonés du benzylidène-camphre, constituant des pigments insolubles dans l'eau, permettait de répondre à l'ensemble de ces exigences et d'obtenir en particulier une excellente protection vis-à-vis du rayonnement ultraviolet, même à faible concentration. L'insolubilité dans l'eau de ces composés contribue par ailleurs à assurer une grande persistance de cette protection même après des bains prolongés.

La présente invention a donc pour objet de nouveaux sels de métaux polyvalents de dérivés sulfonés du benzylidène-camphre répondant à la formule générale suivante :

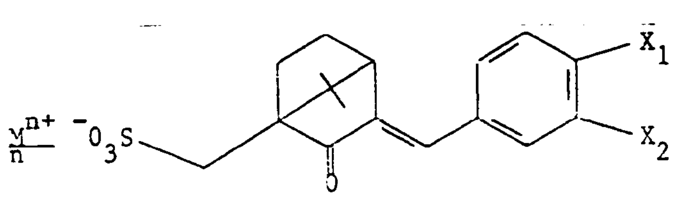

(I)

dans laquelle $M^{n+}$ représente un cation métallique polyvalent dans lequel n est égal à 2, 3 ou 4; $M^{n+}$ désigne de préférence $Ca^{2+}$, $Zn^{2+}$, $Mg^{2+}$, $Ba^{2+}$, $Al^{3+}$ ou $Zr^{4+}$;

l'un des symboles $X_1$ ou $X_2$ désigne un atome d'hydrogène, l'autre désignant l'un des radicaux $Y_1$ ou $Y_2$ suivants :

$-Y_1$ =

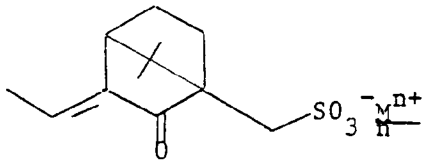

$-Y_2$ =

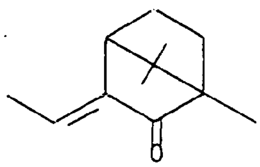

$M^{n+}$ ayant la même signification que ci-dessus.

Les composés (I) selon l'invention sont insolubles dans l'eau mais facilement redispersibles en milieu aqueux.

A titre de composés particulièrement préférés, on peut citer les composés de formule (I) dans lesquels $M^{n+}$ désigne $Ca^{2+}$, $Zn^{2+}$, $Mg^{2+}$, $Al^{3+}$ ou $Zr^{4+}$, $X_2$ désigne un atome d'hydrogène et $X_1$ désigne le radical $Y_1$ ou $Y_2$.

Les composés de formule générale (I) peuvent être préparés en ajoutant un sel ou un hydroxyde de métal polyvalent de formule (II) éventuellement en solution ou en suspension aqueuse

$$(M^{n+})_m(Z^{m-})_n \qquad (II)$$

à une solution aqueuse d'un dérivé de benzylidène-camphre de formule (III)

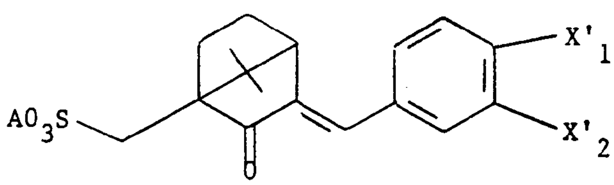

(III)

Dans le composé (II), Z représente un anion minéral ou organique, m est égal à 1 ou 2 et n a la valeur indiquée ci-dessus. Parmi les anions, on peut citer les anions halogénure, en particulier bromure et chlorure, nitrate, acétate, hydroxyle pour les anions monovalents, et les anions carbonate et sulfate pour les anions divalents.

Dans le composé de formule (III),

- A désigne un atome d'hydrogène, un métal alcalin tel que le sodium ou le potassium ou un reste ammonium,
- l'un des symboles $X'_1$ ou $X'_2$ désigne un atome d'hydrogène, l'autre désignant l'un des radicaux $Y'_1$ ou $Y'_2$ suivants :

$-Y'_1$ =

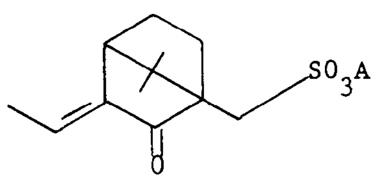

$-Y'_2$ = 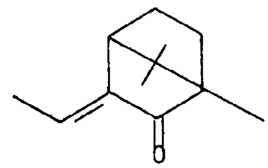

où A a la même signification que ci-dessus.

L'addition du sel de métal polyvalent est effectuée sous agitation. Lorsque le composé de formule (III) est sous forme d'acide sulfonique, le pH du mélange réactionnel peut éventuellement être ajusté au voisinage de la neutralité en cours de réaction par addition d'une solution aqueuse d'un hydroxyde de métal alcalin ou d'ammonium.

Cette réaction peut également être réalisée en inversant l'ordre d'introduction des réactifs.

Le sel de métal polyvalent (II) est de préférence utilisé en quantité stoechiométrique pour salifier le composé de formule (III).

Le composé de formule (I) précipite au cours de la réaction. Il est isolé par filtration puis lavé à l'eau pour éliminer les sels minéraux.

Les composés de formule (III) sont connus et peuvent être préparés selon les modes opératoires décrits dans le brevet FR 2 528 420.

Il est bien entendu que les composés de formule (I) ou (III) peuvent donner lieu à l'isomérie "cis-trans" autour d'une ou plusieurs double(s) liaison(s) et que tous les isomères font partie de l'invention.

La présente invention a également pour objet une composition cosmétique filtrant les rayons UV dans la gamme de longueurs d'onde allant de 280 à 380 nm contenant comme agent de protection contre les rayons ultraviolets, une quantité efficace d'au moins un sel de métal polyvalent de dérivé sulfoné du benzylidène-camphre de formule (I) selon l'invention, dans un milieu cosmétiquement acceptable.

La composition cosmétique selon l'invention, lorsqu'elle est utilisée comme composition destinée à protéger l'épiderme humain contre les rayons ultraviolets, peut se présenter sous les formes les plus diverses habituellement utilisées pour ce type de composition. Elle se présente notamment sous forme de suspension, émulsion telle qu'une crème ou un lait, de gel, de bâtonnet solide, de poudre ou est conditionnée en aérosol.

Elle peut contenir les adjuvants cosmétiques habituellement utilisés dans ce type de composition tels que des épaississants, des adoucissants, des humectants, des tensio-actifs, des conservateurs, des anti-mousses, des parfums, des huiles, des cires, de la lanoline, des propulseurs, des colorants et/ou pigments ayant pour fonction de colorer la composition elle-même ou la peau, ou tout autre ingrédient habituellement utilisé en cosmétique.

Le composé de formule (I) est présent dans des proportions comprises entre 0,25 et 3% en poids par rapport au poids total de la composition.

La présente invention vise également les compositions cosmétiques antisolaires contenant au moins un composé de formule (I) qui peut être associé à d'autres filtres solaires spécifiques du rayonnement UV-B et/ou UV-A et compatibles avec le composé (I) selon l'invention. Dans ce cas, la concentration en composé de formule (I) est comprise entre 0,5 et 10% et la concentration totale en filtres solaires est comprise entre 0,5 et 15 % en poids par rapport au poids total de la composition.

L'invention a également pour objet un procédé de protection de l'épiderme humain contre les rayons UV-A et les rayons UV-B consistant à appliquer sur la peau une quantité efficace d'au moins un composé de formule (I) contenu dans un milieu cosmétiquement acceptable, éventuellement associé à d'autres agents absorbant les rayons UV-A ou UV-B.

L'invention est illustrée par les exemples non limitatifs ci-après.

## EXEMPLES DE PREPARATION

### EXEMPLE 1

Préparation d'un composé de formule générale (1) dans laquelle $M^{n+}$ représente $Mg^{2+}$, $X_1$ est un radical $Y_1$ et $X_2$ est un atome d'hydrogène

On ajoute 3 litres d'eau à 1 kg de solution aqueuse à 30% d'acide téréphtalylidène dicamphosulfonique (0,533 mole). On introduit, sous agitation, 108 g (0,533 mole) de chlorure de magnésium hexahydraté et on maintient l'agitation pendant 90 minutes.

On filtre le mélange réactionnel. Le solide blanc obtenu est lavé à l'eau sous agitation, puis à l'éthanol et

séché sous pression réduite.

On obtient ainsi 260 g de produit attendu sous forme d'une poudre blanche possédant les caractéristiques suivantes :

Point de fusion : > 300°C

Analyse élémentaire : $C_{28}H_{32}O_8S_2Mg$, $5H_2O$

| | C% | H% | O% | S% | Mg% |
|---|---|---|---|---|---|
| Calculé : | 49,81 | 6,27 | 30,81 | 9,49 | 3,60 |
| Trouvé : | 51,14 | 6,57 | 29,51 | 8,67 | 4,11 |

## EXEMPLE 2

Préparation d'un composé de formule générale (I) dans laquelle $M^{n+}$ représente $Zn^{2+}$, $X_1$ est un radical $Y_1$ et $X_2$ est un atome d'hydrogène

On ajoute 700 $cm^3$ d'eau à 200 g de solution aqueuse à 29,5% d'acide téréphtalylidène dicamphosulfonique (0,105 mole). On introduit, sous agitation, 14,2 g (0,105 mole) de chlorure de zinc dihydraté en solution dans 160 $cm^3$ d'eau et on maintient l'agitation pendant 3 heures.

On filtre le mélange réactionnel. Le solide blanc est lavé à l'eau sous agitation et séché sous pression réduite.

On obtient ainsi 57 g de produit attendu sous forme d'une poudre blanche possédant les caractéristiques suivantes :

Point de fusion : > 300°C

Analyse élémentaire : $C_{28}H_{32}O_8S_2Zn$, $6H_2O$

| | C% | H% | O% | S% | Zn% |
|---|---|---|---|---|---|
| Calculé : | 45,78 | 5,99 | 30,52 | 8,72 | 8,86 |
| Trouvé : | 45,30 | 6,03 | 31,09 | 8,68 | 9,11 |

## EXEMPLE 3

Préparation d'un composé de formule générale (I) dans laquelle $M^{n+}$ représente $Al^{3+}$, $X_1$ est un radical $Y_1$ et $X_2$ est un atome d'hydrogène

On ajoute 30 $cm^3$ d'eau à 30 g de solution aqueuse à 29,5% d'acide téréphtalylidène dicamphosulfonique (0,016 mole). On introduit, sous agitation, 2,41 g (0,013 mole) de chlorure d'aluminium trihydraté en solution dans 10 $cm^3$ d'eau. On ajoute 50 $cm^3$ d'eau et on maintient l'agitation pendant 3 heures.

On filtre le mélange réactionnel. Le solide blanc obtenu est lavé à l'eau sous agitation et séché sous pression réduite.

On obtient ainsi 8,4 g de produit attendu sous forme d'une poudre blanche possédant les caractéristiques suivantes :

Point de fusion : > 300°C

Analyse élémentaire : $C_{84}H_{96}O_{24}S_6Al_2$, $22H_2O$

| | C% | H% | O% | S% | Al% |
|---|---|---|---|---|---|
| Calculé : | 47,28 | 6,57 | 34,50 | 9,00 | 2,53 |
| Trouvé : | 46,71 | 6,46 | 31,96 | 8,84 | 2,43 |

EXEMPLE 4

Préparation d'un composé de formule générale (I) dans laquelle $M^{n+}$ représente $Ca^{2+}$, $X_1$ est un radical $Y_2$ et $X_2$ est un atome d'hydrogène

On dissout 4,82 g (0,01 mole) d'acide téréphtalylidène camphre-camphosulfonique dans 100 cm³ d'eau. On introduit, sous agitation, 0,735 g(0,005 mole) de chlorure de calcium dihydraté en solution dans 10 cm³ d'eau et on maintient l'agitation pendant 2 heures.

On filtre le mélange réactionnel. Le solide blanc obtenu est lavé à l'eau sous agitation et séché sous pression réduite.

On obtient ainsi 4,6 g de produit attendu sous forme d'une poudre blanche possédant les caractéristiques suivantes :

Point de fusion : > 300°C

Analyse élémentaire : $C_{56}H_{66}O_{10}S_2Ca$, $4H_2O$

| | C% | H% | O% | S% | Ca% |
|---|---|---|---|---|---|
| Calculé : | 62,51 | 6,88 | 20,84 | 5,95 | 3,72 |
| Trouvé : | 62,80 | 6,86 | 20,79 | 5,85 | 3,67 |

EXEMPLE 5

Préparation d'un composé de formule générale (I) dans laquelle $M^{n+}$ représente $Ca^{2+}$, $X_1$ est un radical $Y_1$ et $X_2$ est un atome d'hydrogène

On ajoute 100 cm³ d'eau à 100 g de solution aqueuse à 29,5% d'acide téréphtalylidène dicamphosulfonique (0,0525 mole). On introduit, sous agitation, 7,7 g (0,0525 mole) de chlorure de calcium dihydraté en solution dans 50 cm³ d'eau et on maintient l'agitation pendant 1 heure.

On filtre le mélange réactionnel. Le solide blanc obtenu est lavé sous agitation et séché sous pression réduite.

On obtient ainsi 27 g de produit attendu sous forme d'une poudre blanche possédant les caractéristiques suivantes :

Point de fusion : > 300°C

Analyse élémentaire : $C_{28}H_{32}O_8S_2Ca$, $4H_2O$

| | C% | H% | O% | S% | Ca% |
|---|---|---|---|---|---|
| Calculé : | 49,92 | 5,94 | 28,53 | 9,50 | 5,94 |
| Trouvé : | 49,61 | 5,99 | 29,06 | 8,90 | 6,44 |

EXEMPLE 6

Préparation d'un composé de formule générale (I) dans laquelle $M^{n+}$ représente $Zr^{4+}$, $X_1$ est un radical $Y_1$, et $X_2$ est un atome d'hydrogène

On ajoute 750 cm³ d'eau à 200 g de solution aqueuse à 30% d'acide téréphtalylidène dicamphosulfonique (0,106 mole).

On introduit sous agitation 12,3 g (0,053 mole) de chlorure de zirconium anhydre dissous dans 925 cm³ d'eau et on maintient l'agitation pendant 1 heure.

On filtre le mélange réactionnel. Le solide blanc est lavé à l'eau puis séché sous pression réduite.

On obtient 46,1 g de produit sous forme d'une poudre blanche présentant les caractéristiques suivantes :

Point de fusion : > 250°C

Rapport en poids entre le carbone et le soufre pour : $C_{56}H_{64}O_{16}S_4Zr$

Calculé : 5,25

Trouvé : 5,20

## EXEMPLES DE FORMULATION

### EXEMPLE A

Lait antisolaire H/E

| | | |
|---|---|---|
| - Composé de l'exemple 1 | | 2,0 g |
| - Paraméthoxycinnamate de 2-éthylhexyle | | 3,0 g |
| - Alcool oléocétylique à 30 moles d'oxyde d'éthylène | | 6,0 g |
| - Alcool stéarylique | | 4,0 g |
| - Benzoate d'alcools $C_{12}$-$C_{15}$ vendu sous la dénomination de "FINSOLV TN" par la Société FINETEX | | 13,0 g |
| - Sorbitol à 70% | | 16,0 g |
| - Conservateur | qs | |
| - Parfum | qs | |
| - Triéthanolamine | qs | pH : 7,0 |
| - Eau déminéralisée | qsp | 100,0 g |

On dissout le p-méthoxycinnamate de 2-éthylhexyle dans la phase grasse qui est chauffée vers 70-75°C. On disperse le composé de l'exemple 1 dans la phase aqueuse qui est chauffée vers 70-75°C.

Sous vive agitation, on ajoute la phase grasse à la phase aqueuse; puis on laisse refroidir sous agitation modérée. Vers 40°C, on ajoute le parfum et le conservateur.

### EXEMPLE B

Crème antisolaire E/H

| | | |
|---|---|---|
| - Composé de l'exemple 5 | | 2,5 g |
| - Composé de l'exemple 2 | | 1,5 g |
| - Paradiméthylaminobenzoate de 2-éthylhexyle | | 6,0 g |
| - Stéarate de magnésium | | 3,5 g |
| - Lanoline hydrogénée vendue sous la dénomination de "HYDROLAN H" par la Société ONYX | | 1,5 g |
| - Lanoline claire | | 4,0 g |
| - Cire d'abeille | | 4,5 g |
| - Sesquioléate de sorbitan | | 4,5 g |
| - Huile de vaseline | | 20,0 g |
| - Octyldodécanol | | 10,0 g |
| - Conservateur | qs | |
| - Parfum | qs | |
| - Triéthanolamine | qs | pH : 6,0 |
| - Eau déminéralisée | qsp | 100,0 g |

Le paradiméthylaminobenzoate de 2-éthylhexyle est dissous dans la phase grasse contenant l'émulsionnant. Les composés des exemples 2 et 5 sont dispersés dans la phase aqueuse.

Les deux phases sont chauffées à 70-75°C et sous vive agitation, on ajoute la phase aqueuse à la phase grasse. On laisse refroidir sous agitation modérée et à 40°C, on ajoute le parfum et le conservateur.

EXEMPLE C

Huile antisolaire épaissie

| | | |
|---|---|---|
| - Composé de l'exemple 3 | | 3,0 g |
| - Paraméthoxycinnamate de 2-éthylhexyle | | 2,5 g |
| - Paradiméthylaminobenzoate de 2-éthylhexyle | | 1,5 g |
| - Huile de colza | | 30,0 g |
| - Silice vendue sous la dénomination d'"AEROSIL R972" par la Société DEGUSSA | | 7,0 g |
| - Tétracyclodiméthylsiloxane | | 10,0 g |
| - Tertiobutylparacrésol | | 0,1 g |
| - Parfum | qs | |
| - Myristate d'isopropyle | qsp | 100,0 g |

On disperse le composé de l'exemple 3 dans la phase grasse contenant les filtres et on ajoute la silice.

EXEMPLE D

Emulsion H/E antisolaire

| | | |
|---|---|---|
| - Composé de l'exemple 2 | | 5,0 g |
| - Alcool cétylstéarylique | | 1,6 g |
| - Alcool cétylstéarylique à 33 moles d'oxyde d'éthylène | | 6,4 g |
| - Mélange de mono et de distéarate de glycérol vendu sous la dénomination de "GELEOL" par la Société GATTEFOSSE | | 3,5 g |
| - Huile de vaseline | | 15,0 g |
| - Propylèneglycol | | 5,0 g |
| - Glycérol | | 15,0 g |
| - Conservateur | qs | |
| - Parfum | qs | |
| - Triéthanolamine | qs | pH : 7,0 |
| - Eau déminéralisée | qsp | 100,0 g |

On chauffe la phase grasse à 70-75°C. On disperse le composé de l'exemple 2 dans la phase aqueuse qui est chauffée à la même température.

Sous vive agitation, on ajoute la phase grasse à la phase aqueuse, puis on laisse refroidir sous agitation modérée. Vers 40°C, on ajoute parfum et conservateur.

EXEMPLE E

Gel aqueux antisolaire

| | | | |
|---|---|---|---|
| - Composé de l'exemple 2 | | | 2,0 g |
| - 2-hydroxy-4-méthoxybenzophénone | | | 0,3 g |
| - Propylèneglycol | | | 15,0 g |
| - Alcool éthylique | | | 5,0 g |
| - Polymère carboxyvinylique vendu sous la dénomination de "CARBOPOL 940" par la Société GOODRICH CHEMICAL | | | 0,4 g |
| - Conservateur | qs | | |
| - Parfum | qs | | |
| - Triéthanolamine | qs | pH : | 6,5 |
| - Eau déminéralisée | qsp | | 100,0 g |

On disperse le composé de l'exemple 2 dans le milieu hydroalcoolique contenant la 2-hydroxy-4-méthoxybenzophénone; on ajoute le Carbopol et enfin la triéthanolamine, le conservateur et le parfum.

EXEMPLE F

Emulsion H/E antisolaire

| | | | |
|---|---|---|---|
| - Composé de l'exemple 3 | | | 1,5 g |
| - Composé de l'exemple 5 | | | 1,5 g |
| - Acide 2-phénylbenzimidazole-5-sulfonique | | | 3,0 g |
| - Mélange 50/50 de monostéarate de glycérol et de stéarate de polyéthylèneglycol à 100 moles d'oxyde d'éthylène vendu sous la dénomination de "SIMULSOL 165" par la Société SEPPIC | | | 8,0 g |
| - Alcool stéarylique | | | 6,0 g |
| - Huile de vaseline | | | 20,0 g |
| - Glycérine | | | 10,0 g |
| - Conservateur | qs | | |
| - Parfum | qs | | |
| - Soude | qs | pH : | 7,0 |
| - Eau déminéralisée | qsp | | 100,0 g |

On chauffe la phase grasse vers 70-75°C. On disperse les composés des exemples 3 et 5 dans la phase aqueuse contenant l'acide 2-phénylbenzimidazole-5-sulfonique, qui est chauffée vers 70-75°C.

Sous vive agitation, on ajoute la phase grasse à la phase aqueuse, puis on laisse refroidir sous agitation modérée. Vers 40°C, on ajoute parfum et conservateur.

EXEMPLE G

Fond de teint anhydre

| | |
|---|---|
| - Composé de l'exemple 1 | 2,0 g |
| - Cire de candelilla | 2,0 g |
| - Ozokérite | 2,0 g |
| - Alcools de lanoline | 2,0 g |
| - Vaseline | 12,0 g |
| - Myristate d'isopropyle | 10,0 g |
| - Stéarate d'isopropyle | 13,0 g |
| - Huile de tournesol | 13,0 g |
| - Isononanoate d'isotridécyle | 20,0 g |
| - Butyl hydroxy toluène | 0,1 g |
| - Talc | 7,0 g |
| - Carbonate de magnésium | 8,0 g |
| - Oxyde de titane | 7,0 g |
| - Oxyde de fer jaune | 1,1 g |
| - Oxyde de fer rouge | 0,7 g |
| - Oxyde de fer noir | 0,1 g |
| | ------- |
| | 100,0 g |

EXEMPLE H

Poudre visage

| | |
|---|---|
| - Composé de l'exemple 2 | 1,0 g |
| - Mica | 20,0 g |
| - Talc | 30,0 g |
| - Amidon de riz modifié | 30,0 g |
| - Kaolin | 2,0 g |
| - Stéarate de zinc | 2,0 g |
| - Oxyde de fer rouge | 0,5 g |
| - Oxyde de fer jaune | 0,8 g |
| - Oxyde de fer noir | 0,1 g |
| - Poudre de Nylon | 8,8 g |
| - Huile de vaseline | 3,0 g |
| - Parfum | 0,8 g |
| - Carbonate de magnésium | 1,0 g |
| | ------- |
| | 100,0 g |

## Revendications

**1.** Sel de métal polyvalent de dérivé sulfoné du benzylidène-camphre ayant la formule générale :

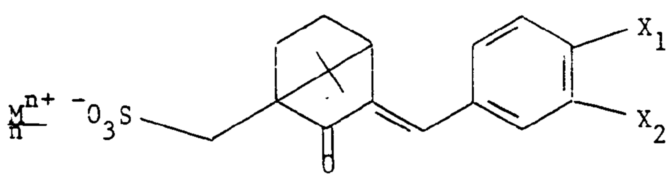

(I)

dans laquelle $M^{n+}$ représente un cation métallique polyvalent dans lequel n est égal à 2, 3 ou 4;

l'un des symboles $X_1$ ou $X_2$ désigne un atome d'hydrogène, l'autre désignant l'un des radicaux $Y_1$ ou $Y_2$ suivants :

$-Y_1$ =

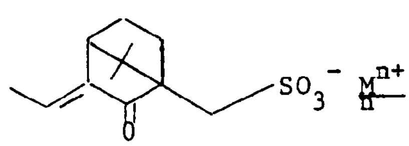

$-Y_2$ =

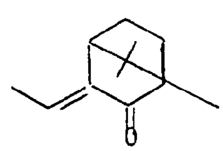

où $M^{n+}$ a la même signification que ci-dessus.

2. Composé selon la revendication 1, caractérisé par le fait que $M^{n+}$ désigne $Ca^{2+}$, $Zn^{2+}$, $Mg^{2+}$, $Ba^{2+}$, $Al^{3+}$ ou $Zr^{4+}$.

3. Composé selon la revendication 1 ou 2, caractérisé par le fait que $X_2$ désigne un atome d'hydrogène et $X_1$ désigne un radical $Y_1$ ou $Y_2$.

4. Composé selon l'une quelconque des revendications 1 à 3, caractérisé par le fait que $M^{n+}$ désigne $Ca^{2+}$, $Zn^{2+}$, $Mg^{2+}$, $Al^{3+}$ ou $Zr^{4+}$, $X_1$ est un radical $Y_1$ ou $Y_2$ et $X_2$ est un atome d'hydrogène.

5. Composition cosmétique filtrant les rayons UV de longueurs d'onde comprises entre 280 et 380 nm, caractérisée par le fait qu'elle contient comme agent de protection contre les rayons UV, une quantité efficace d'au moins un sel de métal polyvalent de dérivé sulfoné du benzylidène-camphre de formule (I) selon la revendication 1, dans un milieu cosmétiquement acceptable.

6. Composition cosmétique filtrante selon la revendication 5, caractérisée par le fait qu'elle contient au moins un composé (I) dans lequel $M^{n+}$ est choisi parmi $Ca^{2+}$, $Zn^{2+}$, $Mg^{2+}$, $Ba^{2+}$, $Al^{3+}$ et $Zr^{4+}$.

7. Composition cosmétique filtrante selon la revendication 6, caractérisée par le fait qu'elle contient au moins un composé (I) dans lequel $M^{n+}$ est choisi parmi $Ca^{2+}$, $Zn^{2+}$, $Mg^{2+}$, $Al^{3+}$ et $Zr^{4+}$, $X_2$ désigne un atome d'hydrogène et $X_1$ est un radical $Y_1$ ou $Y_2$.

8. Composition cosmétique filtrante selon l'une quelconque des revendications 5 à 7, caractérisée par le fait qu'elle se présente sous forme de suspension, émulsion, gel, bâtonnet solide, poudre ou aérosol.

9. Composition cosmétique selon l'une quelconque des revendications 5 à 8, caractérisée par le fait qu'elle contient en outre au moins un adjuvant cosmétique choisi parmi les épaississants, les adoucissants, les humectants, les tensio-actifs, les conservateurs, les anti-mousses, les parfums, les huiles, les cires, la lanoline, les propulseurs, les colorants et pigments.

10. Composition cosmétique selon l'une quelconque des revendications 5 à 9, se présentant sous forme de composition protectrice de l'épiderme humain, caractérisée par le fait que le composé de formule (I) est présent dans des proportions comprises entre 0,25 et 3% en poids par rapport au poids total de la composition.

11. Composition cosmétique selon l'une quelconque des revendications 5 à 9, se présentant sous forme de composition antisolaire, caractérisée par le fait qu'elle contient en plus du composé de formule (I), d'autres filtres solaires filtrant les rayons UV-B ou UV-A.

12. Composition cosmétique antisolaire selon la revendication 11, caractérisée par le fait qu'elle contient de 0,5 à 10% en poids de composé (I) et la concentration totale des filtres solaires est comprise entre 0,5 et 15% en poids.

13. Procédé de protection de l'épiderme humain contre les rayons UV, caractérisé par le fait qu'on applique sur la peau une quantité efficace d'au moins un composé de formule (I) défini dans la revendication 1, contenu dans un milieu cosmétiquement acceptable.

**Patentansprüche**

1. Mehrwertiges Metallsalz eines sulfonierten Derivats von Benzylidencampher mit der allgemeinen Formel:

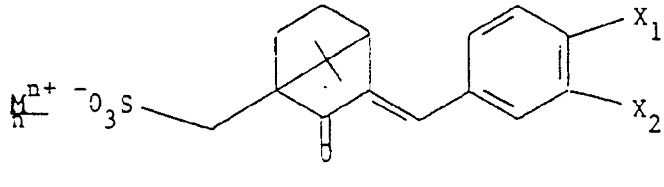

(I)

worin $M^{n+}$ ein mehrwertiges Metallkation darstellt, worin n = 2, 3 oder 4 ist;

eines der Symbole $X_1$ oder $X_2$ ein Wasserstoffatom und das andere einen der Reste $Y_1$ oder $Y_2$ bedeuten:

$-Y_1$ =

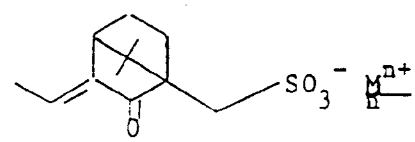

$-Y_2$ =

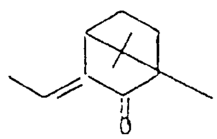

wobei $M^{n+}$ dieselbe Bedeutung wie oben hat.

2. Verbindung gemäß Anspruch 1, dadurch **gekennzeichnet**, daß $M^{n+}$ $Ca^{2+}$, $Zn^{2+}$, $Mg^{2+}$, $Ba^{2+}$, $Al^{3+}$ oder $Zr^{4+}$ bedeutet.

3. Verbindung gemäß Anspruch 1 oder 2, dadurch **gekennzeichnet**, daß $X_2$ ein Wasserstoffatom und $X_1$ einen Rest $Y_1$ oder $Y_2$ bedeuten.

4. Verbindung gemäß eines jeden der Ansprüche 1 bis 3, dadurch **gekennzeichnet,** daß $M^{n+}$ $Ca^{2+}$, $Zn^{2+}$, $Mg^{2+}$, $Al^{3+}$ oder $Zr^{4+}$ bedeutet, $X_1$ einen Rest $Y_1$ oder $Y_2$ und $X_2$ ein Wasserstoffatom darstellen.

5. Kosmetische Zusammensetzung, die die W-Strahlen von Wellenlängen von 280 bis 380 nm filtert, dadurch **gekennzeichnet,** daß sie als Schutzmittel gegen die UV-Strahlen eine wirksame Menge mindestens eines mehrwertigen Metallsalzes des sulfonierten Derivats von Benzylidencampher der Formel (I) gemäß Anspruch 1 in einem kosmetisch verträglichen Medium enthält.

6. Kosmetische Filterzusammensetzung gemäß Anspruch 5, dadurch **gekennzeichnet**, daß sie mindestens eine Verbindung (I) enthält, worin $M^{n+}$ unter $Ca^{2+}$, $Zn^{2+}$, $Mg^{2+}$, $Ba^{2+}$, $Al^{3+}$ und $Zr^{4+}$ ausgewählt ist.

7. Kosmetische Filterzusammensetzung gemäß Anspruch 6, dadurch **gekennzeichnet,** daß sie mindestens eine Verbindung (I) enthält, worin $M^{n+}$ unter $Ca^{2+}$, $Zn^{2+}$, $Mg^{2+}$, $Al^{3+}$ und $Zr^{4+}$ ausgewählt ist, $X_2$ ein Wasserstoffatom und $X_1$ einen Rest $Y_1$ oder $Y_2$ darstellen.

8. Kosmetische Filterzusammensetzung gemäß eines jeden der Ansprüche 5 bis 7, dadurch **gekennzeichnet,** daß sie in form einer Suspension, Emulsion, eines Gels, Feststoffstiftes, Pulvers oder Aerosols vorliegt.

9. Kosmetische Zusammensetzung gemäß eines jeden der Ansprüche 5 bis 8, dadurch **gekennzeichnet,** daß sie zusätzlich mindestens einen kosmetischen Hilfsstoff enthält, ausgewählt aus Verdickungsmitteln, Weichmachern, feuchtigkeitsmitteln, oberflächenaktiven Mitteln, Konservierungsmitteln, Antischaummitteln, Parfüms, Ölen, Wachsen, Lanolin, Treibmitteln, Färbemitteln und Pigmenten.

10. Kosmetische Zusammensetzung gemäß eines jeden der Ansprüche 5 bis 9, die in der form einer die menschliche Haut schützenden Zusammensetzung vorliegt, dadurch **gekennzeichnet,** daß die Verbindung der Formel (I) in Mengenverhältnissen von 0,25 bis 3 Gew.%, bezogen auf Gesamtgewicht der Zusammensetzung, vorliegt.

11. Kosmetische Zusammensetzung gemäß eines jeden der Ansprüche 5 bis 9, die in form einer Zusammensetzung gegen die schädliche Einwirkung der Sonne vorliegt, dadurch **gekennzeichnet**, daß sie zusätzlich zur Verbindung der Formel (I) weitere Sonnenfiltermittel enthält, die die UV-B- oder UV-A-Strahlen filtern.

12. Kosmetische Zusammensetzung gegen die schädliche Einwirkung von Sonne gemäß Anspruch 11, dadurch **gekennzeichnet,** daß sie 0,5 bis 10 Gew.% der Verbindung (I) enthält und die Gesamtkonzentration der Sonnenfiltermittel 0,5 bis 15 Gew.% beträgt.

13. Verfahren zum Schutz der menschlichen Haut gegen die UV-Strahlen, dadurch **gekennzeichnet,** daß man auf die Haut eine wirksame Menge mindestens einer in Anspruch 1 definierten Verbindung der Formel (I), die in einem kosmetisch verträglichen Medium enthalten ist, aufträgt.

## Claims

1. Polyvalent metal salt of a sulphonated derivative of benzylidenecamphor having the general formula:

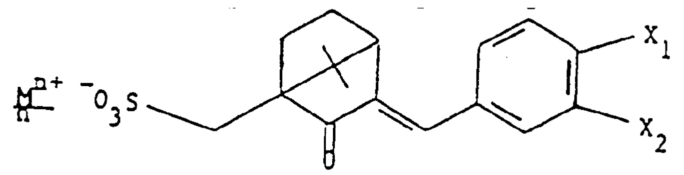

(I)

in which $M^{n+}$ denotes a polyvalent metal carbon in which n is equal to 2, 3 or 4; and one of the symbols $X_1$ or $X_2$ denotes a hydrogen atom, the other denoting one of the following radicals $Y_1$ or $Y_2$:

$- Y_1 =$ 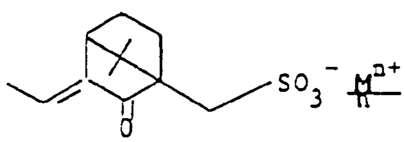

$- Y_2 =$ 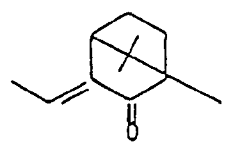

where $M^{n+}$ has the same meaning as above.

2. Compound according to Claim 1, characterized in that $M^{n+}$ denotes $Ca^{2+}$, $Zn^{2+}$, $Mg^{2+}$, $Ba^{2+}$, $Al^{3+}$ or $Zr^{4+}$.

3. Compound according to Claim 1 or 2, characterized in that $X_2$ denotes a hydrogen atom and $X_1$ denotes a radical $Y_1$ or $Y_2$.

4. Compound according to any one of Claims 1 to 3, characterized in that $M^{n+}$ denotes $Ca^{2+}$, $Zn^{2+}$, $Mg^{2+}$, $Al^{3+}$ or $Zr^{4+}$, $X_1$ is a radical $Y_1$ or $Y_2$ and $X_2$ is a hydrogen atom.

5. Cosmetic composition screening out UV rays of wavelengths between 280 and 380 nm, characterized in that it contains, as a protective agent against UV rays, an effective amount of at least one polyvalent metal salt of a sulphonated derivative of benzylidenecamphor of formula (I) according to Claim 1, in a cosmetically acceptable medium.

6. Cosmetic screening composition according to Claim 5, characterized in that it contains at least one compound (I) in which $M^{n+}$ is selected from $Ca^{2+}$, $Zn^{2+}$, $Mg^{2+}$, $Ba^{2+}$, $Al^{3+}$ and $Zr^{4+}$.

7. Cosmetic screening composition according to Claim 6, characterized in that it contains at least one compound (I) in which $M^{n+}$ is selected from $Ca^{2+}$, $Zn^{2+}$, $Mg^{2+}$, $Al^{3+}$ and $Zr^{4+}$, $X_2$ denotes a hydrogen atom and $X_1$ is a radical $Y_1$ or $Y_2$.

8. Cosmetic screening composition according to any one of Claims 5 to 7, characterized in that it is presented in the form of a suspension, emulsion, gel, solid stick, powder or aerosol.

9. Cosmetic composition according to any one of Claims 5 to 8, characterized in that it contains, in addition, at least one cosmetic adjuvant selected from thickeners, emollients, humectants, surfactants, preservatives, antifoams, fragrances, oils, waxes, lanolin, propellants, colourings and pigments.

10. Cosmetic composition according to any one of Claims 5 to 9, presented in the form of a protective composition for the human epidermis, characterized in that the compound of formula (I) is present in proportions of between 0.25 and 3 % by weight relative to the total weight of the composition.

11. Cosmetic composition according to any one of Claims 5 to 9, presented in the form of an antisun composition, characterized in that it contains, in addition to the compound of formula (I), other sunscreens screening out UV-B or UV-A rays.

12. Cosmetic antisun composition according to Claim 11, characterized in that it contains from 0.5 to 10 % by weight of compound (I), and the total concentration of sunscreens is between 0.5 and 15 % by weight.

13. Process for protecting the human epidermis against UV rays, characterized in that an effective amount of at least one compound of formula (I) defined in Claim 1, contained in a cosmetically acceptable medium, is applied to the skin.